# EUROPEAN PATENT APPLICATION

(11) **EP 3 524 183 A1**
(43) Date of publication of application: **14.08.2019**
(21) Application number: 19156766.8
(22) Date of filing: 12.02.2019
(51) Int. Cl.: A61B 17/28, A61B 5/0215, A61B 5/022, A61B 5/026, A61B 17/12, A61B 17/29, A61B 17/122

(54) **CLAMPING DEVICE FOR DETECTING INTERNAL BLOOD PRESSURE**

(30) Priority: 13.02.2018 US 201862629754 P; 11.12.2018 US 201816215770
(71) Applicant: Covidien LP, Mansfield, MA 02048 (US)
(72) Inventor: WILLIAMS, Justin, Southbury, CT Connecticut 06488 (US)
(74) Representative: Maschio, Antonio

(57) **Abstract**

A surgical clamping device that is suitable for detecting internal blood pressure or blood flow includes a first jaw assembly, a second jaw assembly, and an elongate body supporting the first and second jaw assemblies. The second jaw assembly includes an inflatable bladder that defines a cavity. The elongate body includes an inner tube and a piston movably supported within the inner tube. The piston defines a piston chamber within the inner tube and is movable within the inner tube from a retracted position to an advanced position. The cavity of the inflatable bladder communicates with the piston chamber such that movement of the piston from the retracted position towards the advanced position inflates the inflatable bladder.

## Description

### CROSS-REFERENCE TO RELATED APPLICATION

This application claims the benefit of and priority to U.S. Provisional Patent Application No. 62/629,754 filed February 13, 2018, the entire disclosure of which is incorporated by reference herein.

### BACKGROUND

### 1. Technical Description

The present disclosure is directed to surgical clamping devices and, more particularly, to surgical clamping devices for detecting internal blood pressure or blood flow.

### 2. Background of Related Art

In surgical procedures in which tissue is to be stapled, such as for bowel resection, it is desirable to determine blood flow or blood pressure at the stapling site. Good blood flow at the stapling site is important to promote healing of stapled tissue. For example, during normal bowel surgery, a location of a defect or cancer within the bowel is identified. Thereafter, the bowel is mobilized and the defect or cancer is excised leaving in some instances bowel sections that require anastomosis. Prior to performing the anastomosis, a clinician must determine that there is sufficient blood flood at the stapling site to support and promote healing of the anastomosis. If sufficient blood is not present a more suitable location for the anastomosis should be identified.

Currently, blood flow at a stapling site is detected by visually analyzing the tissue or cutting the tissue to see if the tissue bleeds. Blood flow may also be identified using dye to improve visualization of the blood flow. However, the use of dye requires additional equipment and can only be used once before the dye saturates the tissue.

Accordingly, a continuing need exists in the art for a clamping device that is simple in construction and can quickly and easily detect blood flow or blood pressure at an internal surgical or stapling site.

### SUMMARY

One aspect of the present disclosure is directed to a surgical clamping device that includes a first jaw assembly, a second jaw assembly, and an elongate body. The first jaw assembly includes a body having a first clamping portion defining a first tissue engaging surface and a sensor positioned adjacent the first tissue engaging surface. The second jaw assembly includes a body having a second clamping portion defining a second tissue engaging surface and an inflatable bladder defining a cavity. The first jaw assembly is movably supported in relation to the second jaw assembly between an open position and a clamped position. The elongate body includes an inner tube and a piston that is movably supported in a proximal portion of the inner tube. The piston at least partially defines a piston chamber within the inner tube and is movable within the inner tube from a retracted position to an advanced position. A tube has a first end that communicates with the cavity of the inflatable bladder and a second end that communicates with the piston chamber such that movement of the piston from the retracted position towards the advanced position inflates the inflatable bladder.

In some embodiments, the piston chamber includes a fluid and movement of the piston from the retracted position towards the advanced position compresses the fluid within the piston chamber to force the fluid from the piston chamber through the tube and into the inflatable bladder to inflate the bladder.

In certain embodiments, the fluid is air.

In embodiments, the clamping device includes a handle assembly and the elongate body extends distally from the handle assembly.

In some embodiments, the elongate body includes a piston spring that urges the piston towards its retracted position.

In certain embodiments, the elongate body includes a seal that is supported within the inner tube distally of the piston and defines a distal end of the piston chamber.

In embodiments, the seal defines a vent channel that has a first end that communicates with the piston chamber and a second end that communicates with atmosphere.

In some embodiments, the seal defines a valve seat and the elongate body further includes a release plunger having a valve member. The release member is movable from a first position in which the valve member is supported on the valve seat to seal the vent channel to a second position in which the valve member is spaced from the valve seat to open the vent channel.

In certain embodiments, the release plunger is urged to the first position by a plunger spring.

In embodiments, the release plunger includes a valve shaft that extends into the piston chamber.

In some embodiments, the piston is positioned to engage the valve shaft as the piston approaches its advanced position to move the release plunger from the first position to the second position.

In certain embodiments, the piston is configured to releasably engage an actuation rod of the handle assembly.

In embodiments, the first jaw assembly includes a first elongate jaw body and the second jaw assembly includes a second elongate jaw body and each of the first and second elongate jaw bodies include a proximal portion and a distal cam portion.

In some embodiments, the distal cam portion of the first elongate jaw body is secured to the first clamping portion and the distal cam portion of the second elongate jaw body is secured to the second clamping portion.

In certain embodiments, the inner tube is pivotably coupled to the proximal portion of the first and second elongate jaw bodies.

In embodiments, the elongate body includes an outer tube that is movable in relation to the inner tube between retracted and advanced positions and is operably coupled to the distal cam portions of the first and second elongate jaw bodies such that movement of the outer tube in relation to the inner tube causes movement of the first jaw assembly in relation to the second jaw assembly between the open position and the clamped position.

In some embodiments, the elongate body includes a piston aligner that is movably supported in the inner tube and is fixedly coupled to the outer tube such that axial movement of the piston aligner between retracted and advanced positions within the inner tube causes corresponding axial movement of the outer tube about the inner tube between retracted and advanced positions.

In certain embodiments, the inner tube defines elongated slots and the piston aligner is coupled to the outer tube by radial extensions that extend through the elongated slots, such that the elongated slots facilitate axial movement of the outer tube in relation to the inner tube and the first and second jaw assemblies.

In embodiments, the elongate body includes a bushing supported on the outer tube that supports a cam member. The cam member engages the distal cam portions of the first and second elongate jaw bodies such that movement of the outer tube between the retracted and advanced positions causes movement of the first and second jaw assemblies between the open and clamped positions.

In some embodiments, the piston aligner is urged to the advanced position by a clamp spring.

In certain embodiments, the piston aligner is positioned to abut the piston such that the piston aligner and the piston are urged to their advanced positions by the clamp spring. In embodiments, the piston spring is stronger than the clamp spring to retain the piston in the retracted position until the actuation rod of the handle assembly is actuated.

### BRIEF DESCRIPTION OF THE DRAWINGS

Various embodiments of the presently disclosed surgical clamping device are described herein below with reference to the drawings, wherein:
FIG. 1 is a side perspective view of an exemplary embodiment of the presently disclosed surgical clamping device with jaws in an open position;
FIG. 2 is an enlarged view of a distal end of the surgical clamping device shown in FIG. 1;
FIG. 3 is a side perspective view of the surgical clamping device shown in FIG. 1 attached to a manually operated handle assembly;
FIG. 4 is a side perspective view of the surgical clamping device shown in FIG. 1 attached to an electrically powered handle assembly;
FIG. 5 is a side perspective, exploded view of the surgical clamping device shown in FIG. 1;
FIG. 6 is a side perspective view of the surgical clamping device shown in FIG. 1 with the inner and outer tubes shown in phantom;
FIG. 7 is an enlarged view of the indicated area of detail shown in FIG. 6;
FIG. 8 is an enlarged view of the indicated area of detail shown in FIG. 5;
FIG. 9 is an enlarged view of the indicated area of detail shown in FIG. 5;
FIG. 10 is a cross-sectional view taken along section line 10-10 of FIG. 1;
FIG. 11 is an enlarged view of the indicated area of detail shown in FIG. 10;
FIG. 12 is a cross-sectional view taken along section line 12-12 of FIG. 11;
FIG. 13 is a side cross-sectional view of the surgical clamping device shown in FIG. 1 in the actuated, clamped position;
FIG. 14 is an enlarged view of the indicated area of detail shown in FIG. 13;
FIG. 15 is an enlarged view of the indicated area of detail shown in FIG. 14; and
FIG. 16 is a side cross-sectional view of the indicated area of detail shown in FIG. 15 with a piston and release plunger of the surgical clamping device in advanced positions.

### DETAILED DESCRIPTION OF EMBODIMENTS

The presently disclosed surgical clamping device will now be described in detail with reference to the drawings in which like reference numerals designate identical or corresponding elements in each of the several views. However, it is to be understood that the disclosed embodiments are merely exemplary of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

In this description, the term "proximal" is used generally to refer to that portion of the device that is closer to a clinician, while the term "distal" is used generally to refer to that portion of the device that is farther from the clinician. In addition, the term "endoscopic" is used generally used to refer to endoscopic, laparoscopic, arthroscopic, and/or any other procedure conducted through small diameter incision or cannula. Further, the term "clinician" is used generally to refer to medical personnel including doctors, nurses, and support personnel.

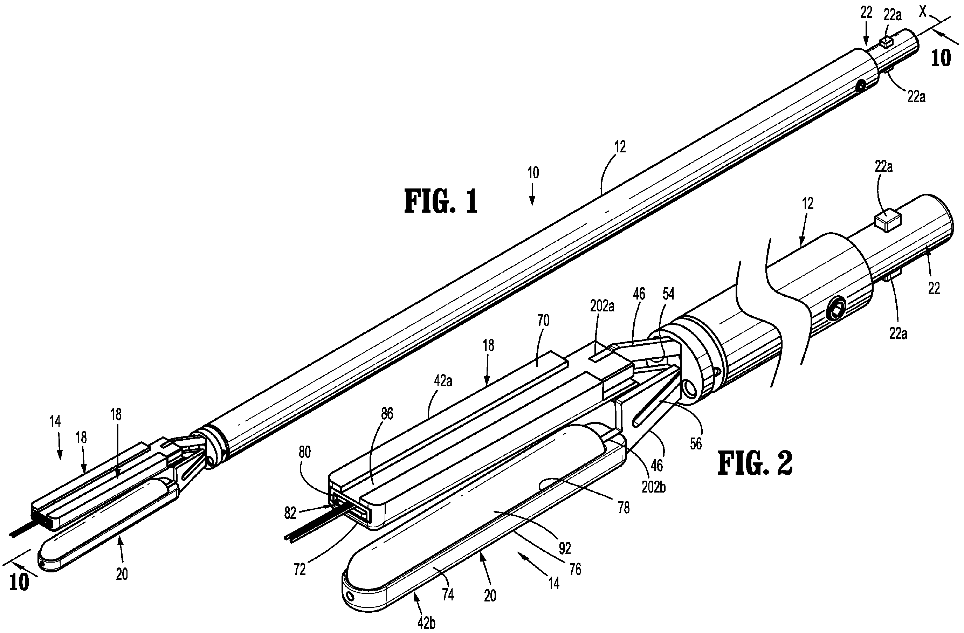

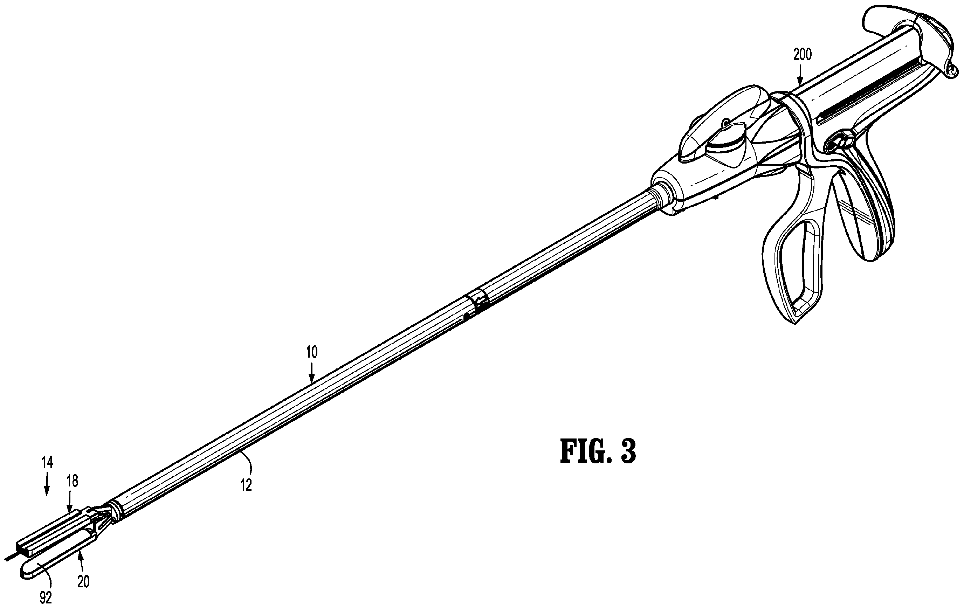

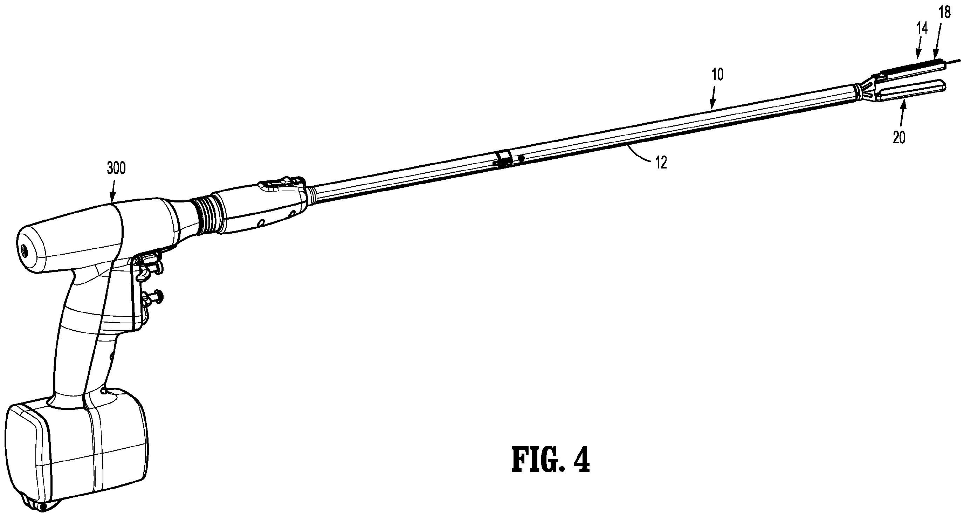

Referring to FIGS. 1-4, the presently disclosed surgical clamping device is shown generally as clamping device 10. The clamping device 10 includes an elongate body 12 defining a longitudinal axis "X" (FIG. 1) and a tool assembly 14 including upper and lower jaw assemblies 18 and 20, respectively. A proximal end of the elongate body 12 includes coupling structure 22 to couple the clamping device 10 to an actuator such as a manually operated handle assembly 200 as shown in FIG. 3 or an electrically actuated handle assembly 300 shown in FIG. 4. It is envisioned that the clamping device 10 can be configured for use with a robotically operated control system. It is also envisioned that the clamping device 10 may include and be integrally formed with either one of the handle assemblies 200, 300. In embodiments, the coupling structure 22 may include projections 22a that form part of a bayonet type coupling assembly. Alternately, it is envisioned that other coupling structures may be used to secure the clamping device 10 to a respective handle assembly 200, 300.

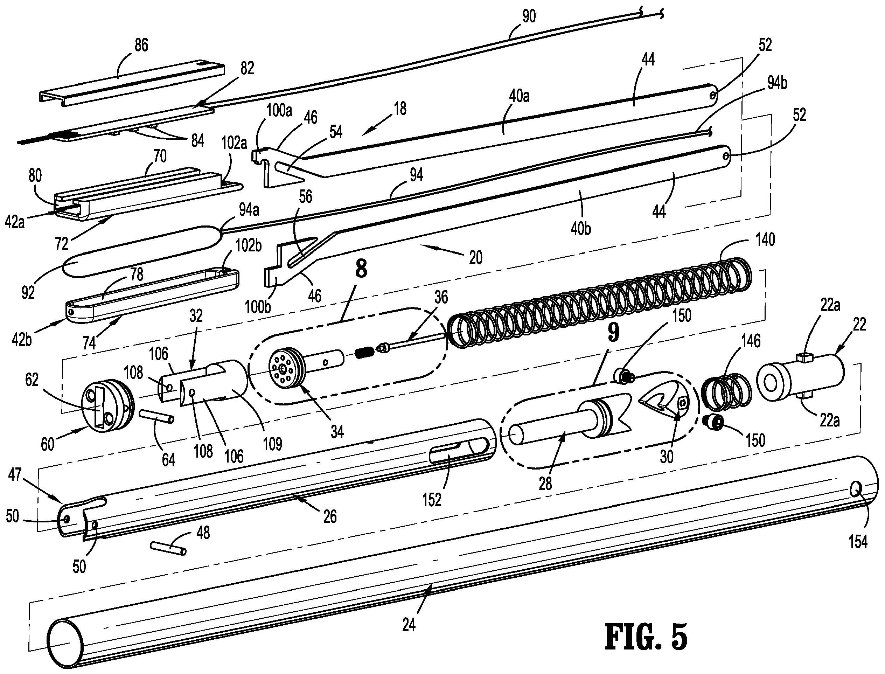

Referring to FIG. 5, the elongated body 12 of the clamping device 10 includes an outer tube 24, an inner tube 26, a piston 28, a piston aligner 30, a yolk 32, a seal 34, and a release plunger 36. The tool assembly 14 including the upper and lower jaw assemblies 18, 20, respectively, has a proximal end supported within the outer tube 24 of elongate body 12 and a distal end that extends from a distal end of the outer tube 12.

Each of the upper and lower jaw assemblies 18, 20 includes an elongate jaw body 40a, 40b, respectively, and a distal clamping portion 42a, 42b, respectively. The distal clamping portion 42a, 42b of the upper and lower jaw assemblies 18, 20 can be integrally formed with the elongate jaw body 40a, 40b or formed separately from the elongated jaw body 12 and subsequently attached thereto using any known fastening technique including welding, pins, or the like.

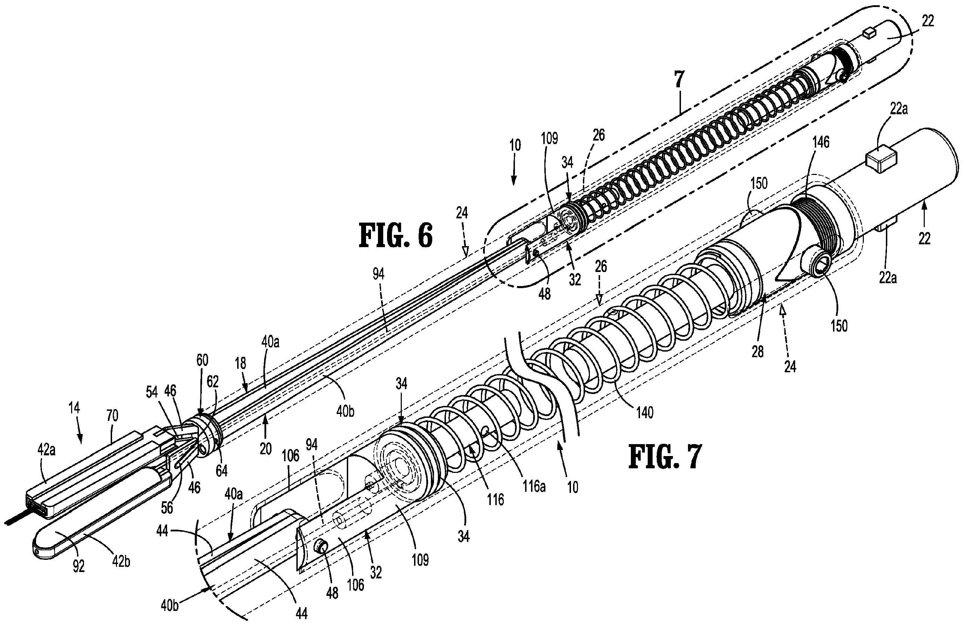

Referring to FIGS. 5-7, each of the elongate jaw bodies 40a, 40b includes a proximal portion 44 and a distal cam portion 46. The proximal portion 44 of each of the upper and lower jaw assemblies 18, 20 is positioned within a distal portion of the outer tube 24 of the elongate body 12 and is pivotally secured to a clevis 47 (FIG. 5) formed on distal end of the inner tube 26 by a pivot pin 48 (FIG. 7). The clevis 47 includes spaced fingers that define openings 50 (FIG. 5) and the proximal portion 44 of each of the elongate jaw bodies 40a, 40b define openings 52 (FIG. 5). The pivot pin 48 extends through openings 50 in the clevis 47 of the inner tube 26 and through the openings 52 in the elongate jaw bodies 40a, 40b of the upper and lower jaw assemblies 18, 20 to pivotally secure the upper and lower jaw assemblies 18, 20 to the inner tube 26 of the elongate body 12.

The distal cam portion 46 of the elongate jaw body 40a of the upper jaw assembly 18 includes a cam slot 54 that diverges outwardly from the longitudinal axis "X" (FIG. 1) in the distal direction. In contrast, the distal cam portion 46 of the elongate jaw body 40b of the lower jaw assembly 20 includes a cam slot 56 that diverges inwardly towards the longitudinal axis "X" in the distal direction.

The distal end of the elongated body 12 supports a bushing 60 that defines a transverse slot 62. The bushing 60 is received within the distal end of the outer tube 24 and supports a pin or cam member 64. The elongate jaw bodies 40a, 40b of the upper and lower jaw assemblies 18, 20 extend through the transverse slot 62 of the bushing 60 such that the cam member 64 is received within the cam slots 54, 56 of the distal cam portions 46 of the elongate jaw bodies 40a, 40b of the upper and lower jaw assemblies 18,20. Due to the configuration of the cam slots 54, 56 of the elongate bodies 40a, 40b, proximal movement of cam member 64 in relation to the upper and lower jaw assemblies 18, 20 causes the upper and lower jaw assemblies 18, 20 to pivot outwardly from the longitudinal axis "X" of the clamping device 10 about the pivot member 48 away from each other to move the tool assembly 14 to an open position. Conversely, distal movement of cam member 64 in relation to the upper and lower jaw assemblies 18, 20 causes the upper and lower jaw assemblies 18, 20 to move inwardly towards the longitudinal axis "X" of the clamping device 10 towards each other to move the tool assembly 14 to a clamped position. In some embodiments, the distance between the pivot member 48 and the distal clamping portions 42a, 42b is of a length such that when the distal clamping portions 42a, 42b are pivoted to the clamped position, the tissue clamping surfaces 72, 76 of the distal clamping portions 42a, 42b remain substantially parallel to each other to effect parallel closure or clamping of the distal clamping portions 42a, 42b. In embodiments, distance between the pivot member 48 and the proximal end of the distal clamping portions 42a, 42b may be greater than 5 inches.

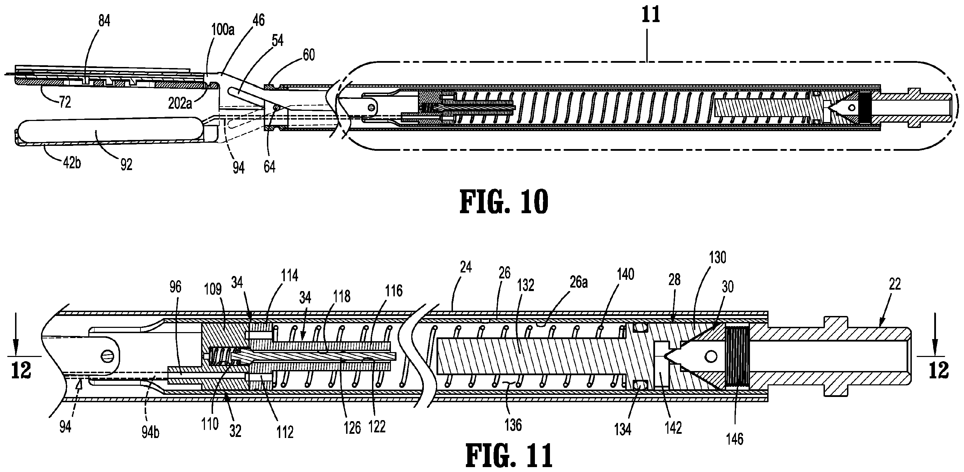

The distal clamping portion 42a of the upper jaw assembly 18 is secured to a distal end of the elongate jaw body 40a and includes a body 70 having a tissue engaging surface 72 (FIG. 5). The distal clamping portion 42b of the lower jaw assembly 20 is secured to a distal end of the elongate body 40b and includes a body 74 (FIG. 5) having a tissue engaging surface 76. The body 74 of the clamping portion 42b defines a hollow 78. In embodiments, the body 70 of the elongate jaw body 40a defines a longitudinally extending recess 80 (FIG. 5) that supports a sensor 82. The sensor 82 includes projections 84 (FIG. 10) that extend through openings in the tissue engaging surface 72 to a position adjacent to the tissue engaging surface 72. In embodiments, a spacer 86 is received in the recess 80 of the body 70 of the upper jaw assembly 18 to secure the sensor 82 in a position to engage tissue adjacent the tissue engaging surface 72 of the body 70. The sensor 82 may be coupled to a controller (not shown) by wires 90 (FIG. 5) that extend from the sensor 82 through the outer tube 24 of the elongate body 12 (FIG. 1) of the clamping device 10.

In embodiments, the hollow 78 of the body 74 of the distal clamping portion 42b of the lower jaw assembly 20 receives an inflatable bladder 92. The inflatable bladder 92 includes a tube 94 (FIG. 5) that extends proximally from the bladder 92. The tube 94 defines a fluid channel that has a distal end 94a that communicates with an internal cavity (not shown) of the bladder 92 and a proximal end 94b that communicates with a port 96 (FIG. 11) defined by the yolk 32 (FIG. 11) as described in further detail below.

The sensor 82 and the inflatable bladder 92 can be provided to identify blood flow or pressure within tissue, e.g., the bowel, which is clamped between the distal clamping portions 42a, 42b of the upper and lower jaw assemblies 18, 20. More specifically, the inflatable bladder 92 can be used to initially occlude blood flow within tissue. Thereafter, the bladder 92 can be vented as described in further detail below, and the sensor 82 can be used to detect the pressure of the blood when blood starts to flow again through the tissue. A controller (not shown) can be provided to receive signals from the sensor 82 via wires 90 to identify blood pressure or blood flow within the tissue. In embodiments, the controller may include visual or audible indicia to provide an indication of the results to a clinician. In embodiments, the sensor 82 can be an LED with a photo diode. Since new flowing blood has more oxygen and thus, a different light absorption rate than stagnate blood, the LED sensor is able to visualize the flow of blood by identifying the light absorption rate of the blood.

Referring to FIG. 5, in embodiments, a distal portion of the each of the elongated jaw bodies 40a, 40b includes an extension 100a, 100b, respectively. The extension 100a of the elongated jaw body 40a may be hook shaped and is configured to be received within a recess 102a (FIG. 2) formed in a proximal portion of the body 70 of the distal clamping portion 42a of the upper jaw assembly 18. The extension 100a can be secured within the recess 102a using any of a variety of fastening techniques including welding, press-fitting and the like. Alternately, the elongated jaw body 40a can be integrally formed with the distal clamping portion 42a.

The extension 100b of the elongated jaw body 40b of the lower jaw assembly 18 is received within a recess 102b (FIG. 4) of the body 74 of the distal clamping portion 42b of the lower jaw assembly 20. As discussed above in regard to the extension 100a, the extension 100b can be secured within the recess 102b of the body 74 of the distal clamping portion 42b using any of a variety of fastening techniques including welding, press-fitting and the like. Alternately, the elongated jaw body 40b can be integrally formed with the distal clamping portion 42b.

Referring again to FIGS. 5-7, the coupling structure 22 is fixedly secured to a proximal portion of the inner tube 26 such that when the coupling structure 22 is secured to one of the handle assemblies 200, 300 (FIGS. 3 and 4), the inner tube 26 is axially fixed in relation to the handle assembly 200, 300. As described above, the proximal portion 44 of each of the elongate jaw bodies 40a, 40b of the upper and lower jaw assemblies 18, 20, is pivotally secured to the clevis 47 formed on the distal end of the inner tube 26 by the pivot pin 48 (FIG. 7). As such, the upper and lower jaw assemblies 18, 20, although rotatable about an axis defined by the pivot pin 48, are axially fixed in relation to the inner tube 26 and the coupling structure 22.

The yolk 32 is fixedly secured within the distal portion of the inner tube 26 and includes spaced fingers 106 that receive the proximal portion 44 of the elongate jaw bodies 40a, 40b. The fingers 106 each define an opening 108 that is aligned with the openings 50 in the distal portion of clevis 47 of the inner tube 26. The pivot pin 48 extends through the openings 50 and 108 to pivotably secure the upper and lower jaw assemblies 18, 20 to the distal portion of the yolk 32 and the inner tube 26.

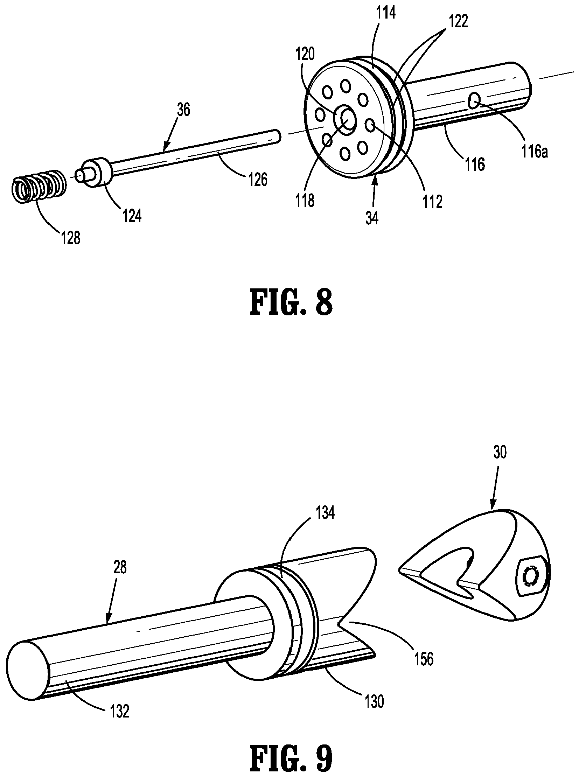

Referring also to FIG. 8, the yolk 32 includes a body 109 that defines the port 96 (FIG. 11) and a central through bore 110. The port 96 has a distal end that communicates with the proximal end 94b (FIG. 5) of the tube 94 and a proximal end that communicates with a bore 112 (FIG. 11) defined in the seal 34. The seal 34 includes a body portion 114 and a central shaft portion 116 that extends proximally from the body portion 114. The central shaft portion 116 and the body portion 114 of the seal 34 define a central vent channel 118 (FIG. 8) that communicates with the central through bore 110 (FIG. 11) of the yolk 32. In embodiments, the central shaft portion 116 defines a bore 116a that communicates with the vent channel 118. In embodiments, the through bore 110 of the yolk 32 has a diameter that is greater than the diameter of the vent channel 118 such that a distal face of the seal 34 defines a valve seat 120 (FIG. 8). The valve seat 120 is positioned to engage the release plunger 36 as described in detail below. In embodiments, the outer surface of the body portion 114 of the seal 34 may include an annular ring or rings 122 to provide a seal between the outer surface of the body portion 114 and an inner wall 26a of the inner tube 26.

Referring to FIGS. 8 and 11, the release plunger 36 includes a valve member 124 and a valve shaft 126. The valve member 124 is positioned within the through bore 110 of the yolk 32 such that the valve shaft 126 extends proximally from the valve member 124, through the vent channel 118 of the seal 34, and projects from the proximal end of the central shaft portion 116 of the seal 34. The release plunger 36 is urged in a proximal direction by a biasing member or plunger spring 128 supported within the central through bore 110 of the yolk 32 to urge the valve member 124 into contact with the valve seat 120 to seal the vent channel 118 of the seal 34.

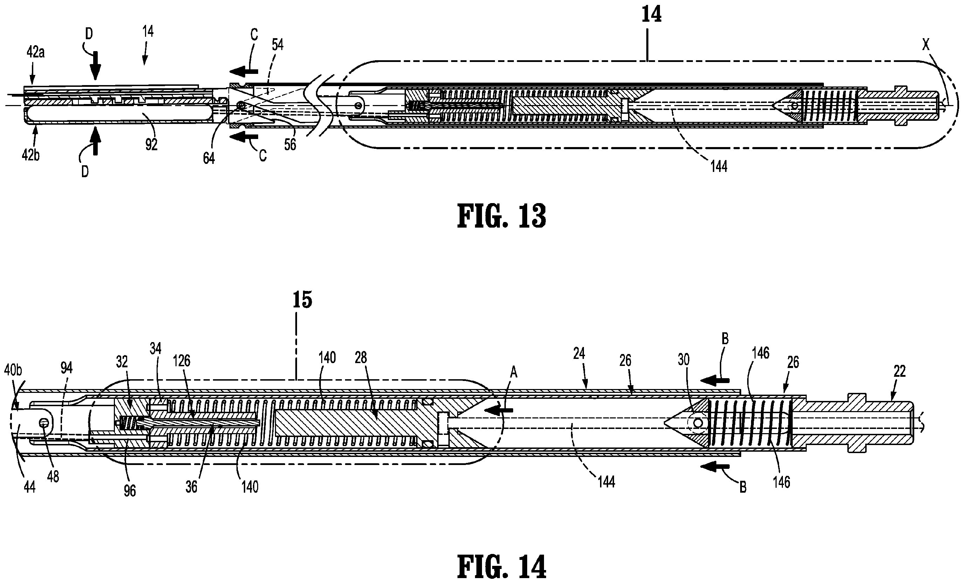

Referring to FIGS. 9-12, the piston 28 is slidably positioned within the inner tube 26 from a retracted position to an advanced position and includes a piston head 130 and a piston shaft 132. The piston head 130 has an outer surface that supports an O-ring 134 that is sealingly engaged with the inner wall 26a of the inner tube 26 such that a piston chamber 136 (FIG. 11) is defined between the seal 34 and the piston 28. The piston head 130 is urged to the retracted position by a biasing member or piston spring 140. The piston head 130 defines a slot 142 that is configured to receive a distal end of a control or actuation rod 144 (FIG. 14) of the handle assembly 200, 300 (FIGS. 3 and 4) such that distal movement of the actuation rod 144 causes movement of the piston 28 from the retracted position to the advanced position against the urging of the piston spring 140. Movement of the piston 28 from the retracted position towards the advanced position causes air to be forced from within the piston chamber 136 through the bore 112 of the yolk 32, through the tube 94, and into the inflatable bladder 92. When the piston 28 approaches its advanced position, the piston shaft 132 of the piston 28 will engage the proximal end of the valve shaft 126 of the release plunger 36 to unseat the valve head 124 of the release plunger 36 from the valve seat 120 of the seal 34 to vent the piston chamber 136 and the inflatable bladder 92 as described in further detail below.

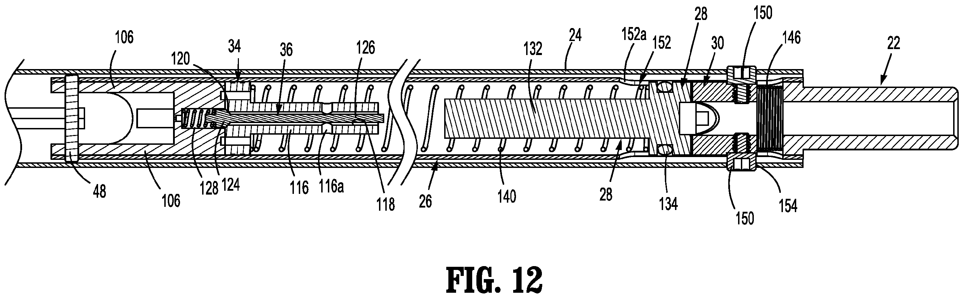

The piston aligner 30 is supported within the proximal portion of the inner tube 26 and has a distal surface that abuts the proximal end of the piston 28 when the piston 28 is in the retracted position (FIG. 11) and a proximal surface that is engaged with a biasing member or clamp spring 146. The piston aligner 30 includes radial extensions 150 (FIG. 12) that are positioned within respective elongated slots 152 (FIG. 12) defined in the proximal portion of the inner tube 26. The radial extensions 150 extend into openings 154 of the outer tube 24 to axially fix the piston aligner 30 to the outer tube 24. In embodiments, the radial extensions 150 may be defined by screws that are threaded into sides of the piston aligner 30 although other protrusion types are envisioned. In embodiments, the piston aligner 30 is wedge shaped and is received in a wedge-shaped groove 156 defined in the proximal surface of the piston 28.

Referring to FIG. 12, the piston spring 140 is stronger than the clamp spring 146 such that prior to actuation of the handle assembly 200, 300 (FIGS. 3 and 4), the piston 28 is urged to the retracted position (FIG. 11). In the retracted position, the radial extensions 150 of the piston aligner 30 are positioned in the proximal end of the elongated slots 152 in the inner tube 26, and the piston shaft 132 of the piston 28 is positioned proximally of the proximal end of the valve shaft 126 of the release plunger 36. As such, the release plunger 36 is urged by the plunger spring 128 proximally to move the valve member 124 into engagement with the valve seat 120 (FIG. 8) to seal the vent channel 118 of the seal 34.

Referring to FIGS. 13 and 14, when the actuation rod 144 is initially moved in the direction indicated by arrow "A" in FIG. 14, the piston 28 is advanced within the inner tube 26 towards its advanced position against the bias of the piston spring 140. As the piston 28 moves within the inner tube 26, the clamp spring 146 urges the piston aligner 30 distally within the inner tube 26 in the direction indicated by arrows "B". Since the piston aligner 30 is secured to the outer tube 24 by the radial extensions 150 (FIG. 12), distal movement of the piston aligner 30 causes corresponding movement of the outer tube 24 about the inner tube 26 until the radial extensions 150 engage the distal end 152a (FIG. 12) of the elongated slot 152 in the inner tube 26. When the radial extensions 150 engage the distal end of the elongated slot 152 of the inner tube 26, further distal movement of the outer tube 24 in relation to the inner tube 26 is prevented.

As the outer tube 24 moves in the direction indicated by arrows "B" in FIG. 13, movement of the cam slots 54, 56 of the each of the elongate jaw bodies 40a, 40b of the upper and lower jaw assemblies 18, 20 in relation to the cam member 64 in the direction indicated by arrows "C" in FIG. 13 causes the distal clamping portions 42a, 42b of the upper and lower jaw assemblies 18, 20 to move inwardly in the direction indicated by arrows "D" in FIG. 13 towards the longitudinal axis "X" (FIG. 13) of the clamping device 10 to the clamped position.

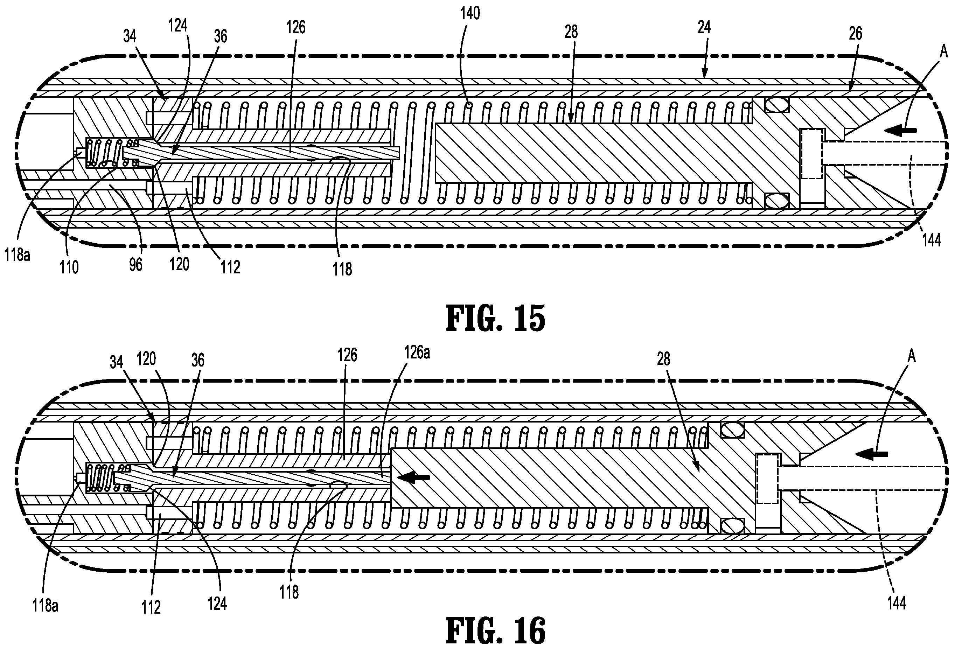

Referring to FIG. 15, as the piston 28 continues to move through the piston chamber 136, the piston 28 forces air through the bore 112 defined in the seal 34, through the port 96 defined in the yolk 32, through the tube 94 (FIG. 14), and into the inflatable bladder 92 to inflate the inflatable bladder 92 (FIG. 13) to inflate the bladder 92.

Referring to FIG. 16, when the piston 28 is advanced to a position in which the piston shaft 132 of the piston 28 engages the proximal end 126a of the valve shaft 126 of the release plunger 36, the valve shaft 126 is urged distally by the piston 28 to move the valve member 124 out of engagement with the valve seat 120 of the seal 34 to open the vent channel 118 of the seal 34. When the vent channel 118 is opened, the piston chamber 136 and the inflatable bladder 92 is vented through the vent channel 118 and the central through bore 110 of the yoke into the distal end of the inner shaft 26 which communicates with atmosphere. In embodiments, the distal end of the vent channel 118 is stepped to define a small diameter orifice 118a that controls the rate of venting of the piston chamber 136 and, thus, venting of the bladder 92.

The presently disclosed clamping device 10 can be used to detect blood flow at a surgical site to identify an appropriate location for an anastomosis. For example, during normal bowel surgery, a location of a defect or cancer within the bowel is identified. Thereafter, the bowel is mobilized and the defect or cancer is excised leaving in some instances bowel sections that require anastomosis. Prior to performing the anastomosis, a clinician must determine that there is sufficient blood flood at the stapling site to support and promote healing of the anastomosis. The presently disclosed clamping device can quickly detect blood flow at the surgical site to identify an appropriate location for creating the anastomosis. More specifically, tissue can be clamped between the distal clamping portions 42a, 42b of the first and second jaw assemblies 18, 20 and the handle assembly 200, 300 (FIGS. 3 and 4) can be actuated to advance the piston 28 and inflate the bladder 92 to initially occlude blood flow through the tissue. When the piston 28 approaches its advanced position, the piston 28 will engage the release plunger 36 to open the vent channel 118. When this occurs, the inflatable bladder 92 will begin to vent. The sensor 82 will then monitor the tissue to determine when blood flow within the tissue begins flowing. Using this determination, the clinician can identify whether or not the tissue has adequate blood flow to support an anastomosis.

Persons skilled in the art will understand that the devices and methods specifically described herein and illustrated in the accompanying drawings are non-limiting exemplary embodiments. It is envisioned that the elements and features illustrated or described in connection with one exemplary embodiment may be combined with the elements and features of another without departing from the scope of the present disclosure. As well, one skilled in the art will appreciate further features and advantages of the disclosure based on the above-described embodiments. Accordingly, the disclosure is not to be limited by what has been particularly shown and described, except as indicated by the appended claims.

The invention may be described by reference to the following numbered paragraphs:-
1. A surgical clamping device comprising:
   a first jaw assembly including a body having a first clamping portion defining a first tissue engaging surface and a sensor positioned adjacent the first tissue engaging surface;
   a second jaw assembly including a body having a second clamping portion defining a second tissue engaging surface and an inflatable bladder defining a cavity, the first jaw assembly being movably supported in relation to the second jaw assembly between an open position and a clamped position;
   an elongate body including an inner tube, and a piston movably supported in a proximal portion of the inner tube, the piston at least partially defining a piston chamber within the inner tube and being movable within the inner tube from a retracted position to an advanced position; and
   a tube having a first end communicating with the cavity of the inflatable bladder and a second end communicating with the piston chamber, wherein movement of the piston from the retracted position towards the advanced position inflates the inflatable bladder.
2. The surgical clamping device of paragraph 1, wherein the piston chamber includes a fluid, and movement of the piston from the retracted position towards the advanced position compresses the fluid within the piston chamber to force the fluid from the piston chamber through the tube and into the inflatable bladder to inflate the bladder.
3. The surgical clamping device of paragraph 2, wherein the fluid is air.
4. The surgical clamping device of paragraph 1, further including a handle assembly, the elongate body extending distally from the handle assembly.
5. The surgical clamping device of paragraph 1, wherein the elongate body includes a piston spring, the piston spring urging the piston towards the retracted position.
6. The surgical clamping device of paragraph 1, wherein the elongate body includes a seal, the seal being supported within the inner tube distally of the piston and defining a distal end of the piston chamber.
7. The surgical clamping device of paragraph 6, wherein the seal defines a vent channel having a first end that communicates with the piston chamber and a second end that communicates with atmosphere.
8. The surgical clamping device of paragraph 7, wherein the seal defines a valve seat and the elongate body further includes a release plunger having a valve member, the release member being movable from a first position in which the valve member is supported on the valve seat to seal the vent channel to a second position in which the valve member is spaced from the valve seat to open the vent channel.
9. The surgical clamping device of paragraph 8, wherein the release plunger is urged to the first position by a plunger spring.
10. The surgical clamping device of paragraph 9, wherein the release plunger includes a valve shaft that extends into the piston chamber.
11. The surgical clamping device of paragraph 10, wherein the piston is positioned to engage the valve shaft as the piston approaches the advanced position to move the release plunger from the first position to the second position.
12. The surgical clamping device of paragraph 4, wherein the piston is configured to engage an actuation rod of the handle assembly.
13. The surgical clamping device of paragraph 1, wherein the first jaw assembly includes a first elongate jaw body and the second jaw assembly includes a second elongate jaw body, each of the first and second elongate jaw bodies including a proximal portion and a distal cam portion, the distal cam portion of the first elongate jaw body being secured to the first clamping portion and the distal cam portion of the second elongate jaw body being secured to the second clamping portion.
14. The surgical clamping device of paragraph 13, wherein the inner tube is pivotably coupled to the proximal portion of the first and second elongate jaw bodies.
15. The surgical clamping device of paragraph 14, wherein the elongate body includes an outer tube, the outer tube being movable in relation to the inner tube between retracted and advanced positions and being operably coupled to the distal cam portions of the first and second elongate jaw bodies such that movement of the outer tube in relation to the inner tube causes movement of the first jaw assembly in relation to the second jaw assembly between the open position and the clamped position.
16. The surgical clamping device of paragraph 15, wherein the elongate body includes a piston aligner movably supported in the inner tube, the piston aligner being fixedly coupled to the outer tube such that axial movement of the piston aligner between retracted and advanced positions within the inner tube causes corresponding axial movement of the outer tube about the inner tube between retracted and advanced positions.
17. The surgical clamping device of paragraph 16, wherein the inner tube defines elongated slots and the piston aligner is coupled to the outer tube by radial extensions that extend through the elongated slots, wherein the elongated slots facilitate axial movement of the outer tube in relation to the inner tube and the first and second jaw assemblies.
18. The surgical clamping device of paragraph 17, wherein the elongate body further includes a bushing supported on the outer tube, the bushing supporting a cam member, the cam member being engaged with the distal cam portions of the first and second elongate jaw bodies such that movement of the outer tube between retracted and advanced positions causes movement of the first and second jaw assemblies between the open and clamped positions.
19. The surgical clamping device of paragraph 18, wherein the piston aligner is urged to the advanced position by a clamp spring.
20. The surgical clamping device of paragraph 19, wherein the piston aligner is positioned to abut the piston such that the piston aligner and the piston are urged to their advanced positions by the clamp spring, and wherein the piston is urged to its retracted position by a piston spring, the piston spring being stronger than the clamp spring to retain the piston in the retracted position.

## Claims

1. A surgical clamping device comprising:
a first jaw assembly including a body having a first clamping portion defining a first tissue engaging surface and a sensor positioned adjacent the first tissue engaging surface;
a second jaw assembly including a body having a second clamping portion defining a second tissue engaging surface and an inflatable bladder defining a cavity, the first jaw assembly being movably supported in relation to the second jaw assembly between an open position and a clamped position;
an elongate body including an inner tube, and a piston movably supported in a proximal portion of the inner tube, the piston at least partially defining a piston chamber within the inner tube and being movable within the inner tube from a retracted position to an advanced position; and
a tube having a first end communicating with the cavity of the inflatable bladder and a second end communicating with the piston chamber, wherein movement of the piston from the retracted position towards the advanced position inflates the inflatable bladder.

2. The surgical clamping device of claim 1, wherein the piston chamber includes a fluid, and movement of the piston from the retracted position towards the advanced position compresses the fluid within the piston chamber to force the fluid from the piston chamber through the tube and into the inflatable bladder to inflate the bladder; preferably wherein the fluid is air.

3. The surgical clamping device of claim 1 or claim 2, further including a handle assembly, the elongate body extending distally from the handle assembly.

4. The surgical clamping device of any preceding claim wherein the elongate body includes a piston spring, the piston spring urging the piston towards the retracted position; and/or wherein the elongate body includes a seal, the seal being supported within the inner tube distally of the piston and defining a distal end of the piston chamber; preferably wherein the seal defines a vent channel having a first end that communicates with the piston chamber and a second end that communicates with atmosphere.

5. The surgical clamping device of claim 4, wherein the seal defines a valve seat and the elongate body further includes a release plunger having a valve member, the release member being movable from a first position in which the valve member is supported on the valve seat to seal the vent channel to a second position in which the valve member is spaced from the valve seat to open the vent channel; preferably wherein the release plunger is urged to the first position by a plunger spring.

6. The surgical clamping device of claim 5, wherein the release plunger includes a valve shaft that extends into the piston chamber; preferably wherein the piston is positioned to engage the valve shaft as the piston approaches the advanced position to move the release plunger from the first position to the second position.

7. The surgical clamping device of claim 4, wherein the piston is configured to engage an actuation rod of the handle assembly; and/or wherein the first jaw assembly includes a first elongate jaw body and the second jaw assembly includes a second elongate jaw body, each of the first and second elongate jaw bodies including a proximal portion and a distal cam portion, the distal cam portion of the first elongate jaw body being secured to the first clamping portion and the distal cam portion of the second elongate jaw body being secured to the second clamping portion.

8. The surgical clamping device of claim 7, wherein the inner tube is pivotably coupled to the proximal portion of the first and second elongate jaw bodies.

9. The surgical clamping device of claim 8, wherein the elongate body includes an outer tube, the outer tube being movable in relation to the inner tube between retracted and advanced positions and being operably coupled to the distal cam portions of the first and second elongate jaw bodies such that movement of the outer tube in relation to the inner tube causes movement of the first jaw assembly in relation to the second jaw assembly between the open position and the clamped position.

10. The surgical clamping device of claim 9, wherein the elongate body includes a piston aligner movably supported in the inner tube, the piston aligner being fixedly coupled to the outer tube such that axial movement of the piston aligner between retracted and advanced positions within the inner tube causes corresponding axial movement of the outer tube about the inner tube between retracted and advanced positions.

11. The surgical clamping device of claim 10, wherein the inner tube defines elongated slots and the piston aligner is coupled to the outer tube by radial extensions that extend through the elongated slots, wherein the elongated slots facilitate axial movement of the outer tube in relation to the inner tube and the first and second jaw assemblies.

12. The surgical clamping device of claim 11, wherein the elongate body further includes a bushing supported on the outer tube, the bushing supporting a cam member, the cam member being engaged with the distal cam portions of the first and second elongate jaw bodies such that movement of the outer tube between retracted and advanced positions causes movement of the first and second jaw assemblies between the open and clamped positions.

13. The surgical clamping device of claim 12, wherein the piston aligner is urged to the advanced position by a clamp spring.

14. The surgical clamping device of claim 13, wherein the piston aligner is positioned to abut the piston such that the piston aligner and the piston are urged to their advanced positions by the clamp spring, and wherein the piston is urged to its retracted position by a piston spring, the piston spring being stronger than the clamp spring to retain the piston in the retracted position.
